# EUROPEAN PATENT APPLICATION

(11) **EP 2 742 912 A2**
(43) Date of publication of application: **18.06.2014**
(21) Application number: 12823906.8
(22) Date of filing: 06.08.2012
(51) Int. Cl.: A61F 2/24, A61B 17/00, A61M 25/01, A61F 2/06

(54) **DEVICE FOR MITRAL VALVE MEMBRANE CERCLAGE PROCEDURE**

(30) Priority: 12.08.2011 KR 20110080392
(71) Applicant: Pusan National University Industry-University Cooperation Foundation, Busan 609-735 (KR)
(72) Inventor: KIM, June-Hong, Busan 613-010 (KR)
(74) Representative: Ferreccio, Rinaldo
(86) International application number: PCT/KR2012/006237
(87) International publication number: WO 2013/024998

(57) **Abstract**

A mitral valve cerclage annuloplasty apparatus, including: a tissue protective tube (20) having a hollow cylindrical coronary sinus tube (22), a hollow cylindrical tricuspid valve tube (24) and a stem part (26) formed by extensions of both the coronary sinus tube and the tricuspid valve tube, with catching depressions (28) formed on the stem part (26); and a cap (30) into which the tissue protective tube is inserted, with a blocking plate (31) provided on a top surface of the cap (30), at least two through holes (32) formed in the blocking plate (31), and catching protrusions (35) formed on the cap (30) so as to correspond to the catching depressions, wherein the catching depressions (28) and the catching protrusions (35) are configured to allow easy separation of the tissue protective tube from the cap but to resist insertion of the tissue protective tube into the cap.

## Description

### [Technical Field]

The present invention relates, in general, to a mitral valve cerclage annuloplasty apparatus which is used during mitral valve cerclage annuloplasty performed on a mitral valve cerclage patient with mitral regurgitation, and which functions to safely and efficiently maintain a final appropriate tension of a cerclage suture required during mitral valve cerclage annuloplasty.

### [Background Art]

The heart is the center of the human circulatory system that pumps blood through our body. It is a muscle that pumps the blood only in one direction. In order for the heart to effectively maintain this unidirectional flow of blood, it must have properly functional valves that prevent back flow through its system, or regurgitation. The heart is divided into four chambers: right and left atrium, and right and left ventricles. The four chambers are connected to the aorta, inferior and superior vena cava, pulmonary artery, and pulmonary veins.

The mitral valve (MV) separates left atrium from left ventricle while the tricuspid valve (TV) separates right atrium from the right ventricle. The aortic valve (AV) is located between the left ventricle and the aorta while the pulmonary valve (PV) is located between the right ventricle and the pulmonary artery.

Generally, valves should open and close completely with every heart beat or contraction. Incomplete opening or closing of the valves causes improper flow of blood. Valvular diseases are divided into two categories, regurgitation and stenosis. Regurgitation is a failure of a valve to close completely and stenosis is a failure of a valve to open completely.

Mitral valve regurgitation (MVR) is a common cardiac valve disorder that is caused by incomplete closure of mitral valve (MV). MV is located between the left atrium and the left ventricle. Over time, MVR places a burden on the heart and worsens its ability to pump blood properly. Such stress on the heart will ultimately lead to heart failure.

Traditional treatment for worsening MVR requires open heart surgery with sternotomy or thoracotomy with cardiac arrest and cardio-pulmonary bypass. Once the chest is open and access to heart is gained, MV is either repaired or replaced using an artificial valve. Although very effective, this open-heart procedure is a high risk surgery accompanied by substantial morbidity and prolonged convalescence. Mortality due to surgery itself can be as high as 5%. As a result, the procedure often is not offered to patients who are insufficiently symptomatic to justify the surgical risk and morbidity, or to patients with substantial co-morbidity. It is reserved only for those with severe symptomatic MVR.

This high morbidity rate of open heart surgery has motivated further research into developing a safer and less risky alternative to repair MVR. Much of the research involves use of cardiac catheterization. Recently, the inventor of the present invention presented a thesis regarding "mitral valve cerclage coronary sinus annuloplasty (MVA" showing outstanding results of MVR treatment through applying circular pressure around the mitral annulus (MA). This thesis has been filed through PCT as an international patent application (PCT application number PCT/US2007/023876) and has been published (International publication number WO2008/060553).

The aforementioned thesis and published patent application disclosed the mitral cerclage coronary annuloplasty (MVA) procedure. Briefly explained, a catheter is placed in coronary sinus after accessing the right atrium through the jugular vein, and then a cerclage suture is passed through the proximal septal vein. This cerclage suture can easily pass through right ventricular outflow tract (RVOT), and this inventor defines this technique as "simple mitral cerclage annuloplasty." Then, the cerclage suture can be easily pulled into the right atrium thus placing the cerclage suture circumferentially around the mitral annulus. Once positioned, tension is applied to the cerclage suture, thereby tightening the mitral valve. This brings together the two lobes of MV so that they are approximated and reduce the size of its incomplete closure. This procedure can theoretically obtain very similar results to those that conventional surgeries can obtain by directly tightening the mitral annulus, and shows an immediate reduction of MVR.

However, the technology disclosed in the above-mentioned PCT application has following technical problems to be solved. First, a tension locking device is required in order to be able to apply proper tension to the cerclage suture and to maintain the tension. Second, since this tension is maintained with a very fine cerclage suture (i.e., 0.014" nylon cerclage was used in the research, but the thickness may be changed), it can cause damage to the neighboring cardiac tissues at points where the suture comes into contact with the tissues and exerts pressure.

In an effort to solve the above-mentioned problems, the inventor of the present invention filed a patent application (Korean Patent Application No. 2009-0080708, field on August 28, 2009 and entitled "Device for delivering optimal tension safely and effectively in cerclage annuloplasty procedure") to KIPO.

The invention disclosed in the above-mentioned patent application was also filed to USPTO, etc. in addition to KIPO.

In the Korean patent application, the inventor proposed a technology in which a mitral valve cerclage annuloplasty procedure is performed by covering a cerclage suture with both a coronary sinus tube (CS Tube) and a tricuspid valve tube (TV Tube) so as to protect cardiac tissues from cerclage suture-caused damage (in other words, by protecting the cardiac tissues from the cerclage suture-caused damage using a tissue protective device) and by delivering a knot formed by knotting ends of the cerclage suture together to an upper end of the tissue protective device using a knot delivery device.

However, the technology disclosed in the Korean patent application is problematic in that, although the knot formed by knotting the ends of the cerclage suture is delivered to the upper end of the tissue protective device using the knot delivery device, a space remains between the knot and the upper end of the tissue protective device, so the cerclage suture may not be appropriately tensioned, but may be loosened.

When the cerclage suture is loosened during mitral valve cerclage annuloplasty procedure as described above (in other words, when the tension of the cerclage suture is reduced), the operational effects in which the mitral annulus can be tightened using the tension of the cerclage suture are reduced, so the cerclage annuloplasty effects of reducing mitral valve regurgitation (MVR) may be reduced.

### [Disclosure]

### [Technical Problem]

Accordingly, the present invention has been made keeping in mind the above problems occurring in the prior art, and an object of the present invention is to provide a mitral valve cerclage annuloplasty apparatus which is used during mitral valve cerclage annuloplasty performed on mitral valve cerclage patients with mitral regurgitation and can continuously maintain predetermined tension of a cerclage suture, thereby continuously and effectively tightening the mitral annulus using the tensioned cerclage suture and increasing the success rate of mitral valve cerclage annuloplasty.

Another object of the present invention is to provide the present invention is to provide a mitral valve cerclage annuloplasty apparatus which can maintain a desired level of tension of the cerclage suture constantly for a lengthy period of time by an easy and simple method.

### [Technical Solution]

In order to accomplish the above objectives, in an aspect, the present invention provides a mitral valve cerclage annuloplasty apparatus, including: a tissue protective tube 20 including a coronary sinus tube 22 that is a hollow cylindrical tube used to protect coronary sinus tissues, a tricuspid valve tube 24 that is a hollow cylindrical tube used to commonly protect tricuspid valve tissues and interventricular septum tissues, and a stem part 26 which is formed by respective extensions of both the coronary sinus tube and the tricuspid valve tube and in which the coronary sinus tube and the tricuspid valve tube are integrated with each other, with catching depressions 28 formed on an outer circumferential surface of the stem part 26; and
a cap 30 having a hollow structure so as to allow the tissue protective tube to be inserted into the cap, with a blocking plate 31 provided on a top surface of the cap 30, at least two through holes 32 formed in the blocking plate 31, and catching protrusions 35 formed on the cap 30 so as to correspond to the catching depressions of the tissue protective tube, wherein
the catching depressions 28 of the tissue protective tube and the catching protrusions 35 of the cap are configured to allow easy separation of the tissue protective tube from the cap but to resist insertion of the tissue protective tube into the cap.

In an embodiment, the top surface of the cap 30 may be provided with a hook ring 34 for hooking a line.

In an embodiment, the catching depressions of the tissue protective tube or the catching protrusions 35 of the cap may be configured to be tooth-shaped.

In another embodiment, the catching depressions of the tissue protective tube may be configured to be tooth-shaped and the catching protrusions of the cap may be formed by regularly spaced grooves.

In another embodiment, the cap 30 may include a body part 38 and a plug part 36 that is placed on an upper end of the body part, with an insert step 36a provided on a lower end of the plug part 36 so as to be inserted into the body part.

In a further embodiment, an outer circumferential surface of the insert step of the plug part may be provided with threads 36b and an inner circumferential surface of the body part may be provided with threads 38b corresponding to the threads of the plug part.

In yet another embodiment, the catching depressions 28 of the tissue protective tube 20 may be formed on opposite surfaces of the tissue protective tube 20 in lengthwise directions of the tissue protective tube.

### [Advantageous Effects]

As described above, the mitral valve cerclage annuloplasty apparatus of the present invention is advantageous in that the cap is added to the tissue protective tube, so, when the cap is raised upward relative to the tissue protective tube, the knot of the cerclage suture is blocked by the blocking plate of the cap, thereby continuously maintaining predetermined tension of the cerclage suture and continuing the effects of tightening the mitral annulus using the tensioned cerclage suture, and increasing the success rate of mitral valve cerclage annuloplasty.

### [Description of Drawings]

FIGS. 1 and 2 are perspective views of a mitral valve cerclage annuloplasty apparatus according to a preferred embodiment of the present invention, in which FIG. 1 illustrates a state in which a tissue protective tube and a cap are separated from each other, and FIG. 2 illustrates a state in which the tissue protective tube and the cap are combined with each other into a single body;
FIGS. 3 to 5 are views illustrating mitral valve cerclage annuloplasty performed using the mitral valve cerclage annuloplasty apparatus according to the preferred embodiment of the present invention, in which FIG. 3 illustrates a state in which a knot is formed in a cerclage suture, FIG. 4 illustrates a state in which the cap is raised upward from the state of FIG. 3 using a drawing line and the knot is blocked by an blocking plate of the cap, and FIG. 5 illustrates a state in which a portion of the cerclage suture is cut at a location above the knot and the drawing line is removed;
FIG. 6 is a perspective view of the mitral valve cerclage annuloplasty apparatus according to the preferred embodiment of the present invention, in which an example of methods of combining the tissue protective tube with the cap is illustrated;
FIG. 7 is a perspective view of a mitral valve cerclage annuloplasty apparatus according to another preferred embodiment of the present invention, in which the shape of the cap is altered;
FIG. 8 is a perspective view of a mitral valve cerclage annuloplasty apparatus according to a further preferred embodiment of the present invention, in which the shape of the cap is further altered;
FIG. 9 is a perspective view of a mitral valve cerclage annuloplasty apparatus according to still another preferred embodiment of the present invention, in which the shape of catching depressions of the tissue protective tube is altered; and
FIG. 10 is a perspective view of a mitral valve cerclage annuloplasty apparatus according to yet another preferred embodiment of the present invention, in which the shape of catching protrusions of the cap is altered.

### [Best Mode]

Hereinbelow, mitral valve cerclage annuloplasty apparatuses according to preferred embodiments of the present invention will be described in detail with reference to the accompanying drawings.

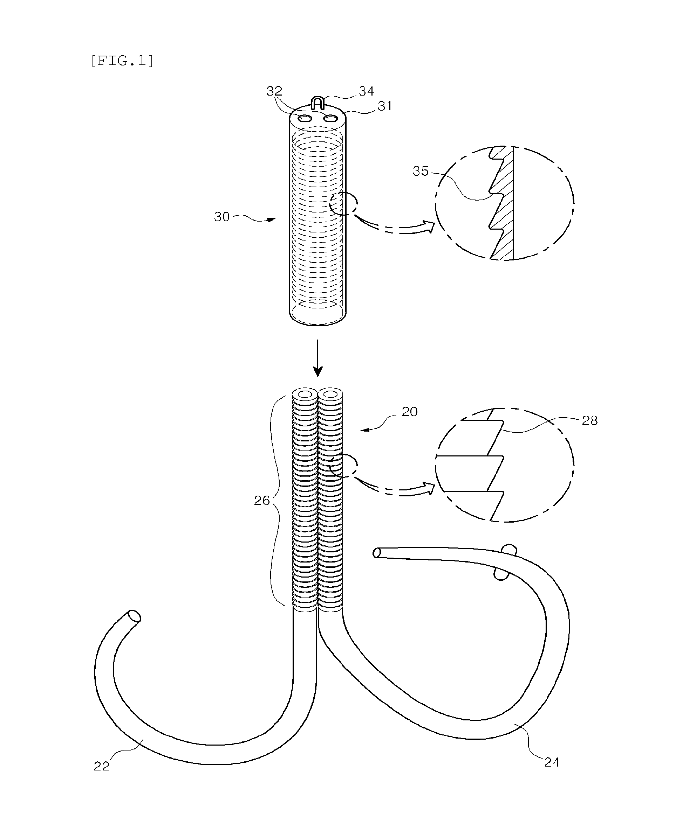

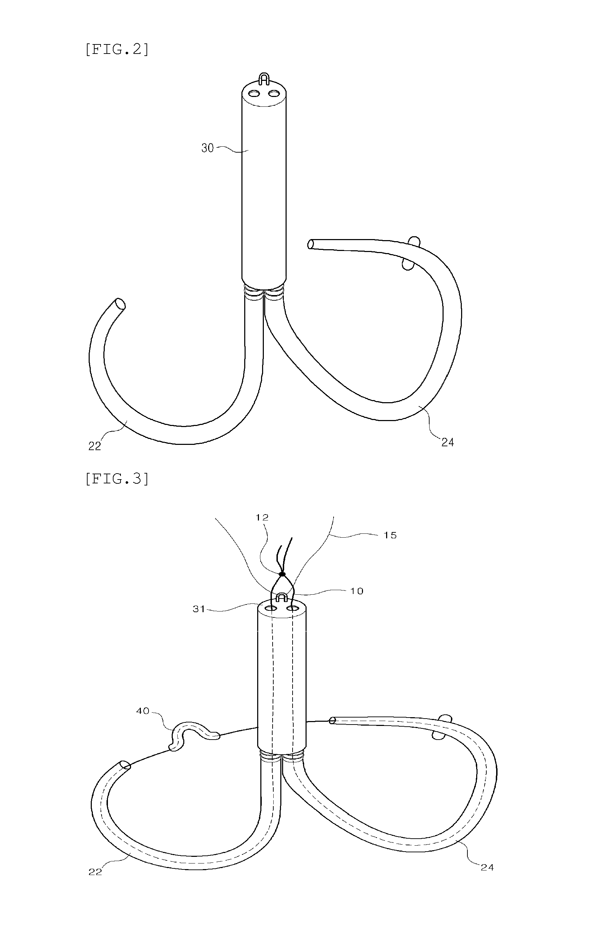

FIGS. 1 and 2 are perspective views of a mitral valve cerclage annuloplasty apparatus according to a preferred embodiment of the present invention, in which FIG. 1 illustrates a state in which a tissue protective tube 20 and a cap 30 are separated from each other, and FIG. 2 illustrates a state in which the tissue protective tube 20 and the cap 30 are assembled with each other into a single body.

With reference to FIGS. 1 and 2, the mitral valve cerclage annuloplasty apparatus of the present invention includes a tissue protective tube 20, and a cap 30 that covers the tissue protective tube 20. When compared to the technology disclosed in the related art patent application (Korean Patent Application No. 2009-0080708) filed by the inventor of the present invention, the technology of this invention is different from the related art technology in that catching depressions 28 are formed on the outer circumferential surface of the tissue protective tube 20 of this invention.

In other words, the tissue protective tube 20 that functions to prevent a cerclage suture from causing damage to cardiac tissues includes a coronary sinus tube 22 that is a hollow cylindrical tube used to protect coronary sinus (CS) tissues, a tricuspid valve tube 24 that is a hollow cylindrical tube used to commonly protect tricuspid valve (TV) tissues and interventricular septum (IVS) tissues, and a stem part 26 which is formed by respective extensions of both the coronary sinus tube and the tricuspid valve tube and in which the coronary sinus tube and the tricuspid valve tube are integrated with each other, with catching depressions 28 formed on the outer circumferential surface of the stem part 26.

The cap 30 has a hollow structure, so it is possible to insert the tissue protective tube 20 into the cap 30. Catching protrusions 35 corresponding to the catching depressions 28 of the tissue protective tube 20 are formed on the inner circumferential surface of the cap 30.

Here, the catching depressions 28 of the tissue protective tube and the catching protrusions 35 of the cap are configured to allow easy separation of the tissue protective tube from the cap but to resist insertion of the tissue protective tube into the cap. The above-mentioned specified configuration of both the catching depressions 28 and the catching protrusions 35 is intended to maintain a predetermined tension of the cerclage suture when raising the cap 30 upward after making a knot in the cerclage suture during mitral valve cerclage annuloplasty.

To this end, as shown in the drawings, it is preferred that the catching depressions 28 of the tissue protective tube 20 and/or the catching protrusions 35 of the cap 30 are configured to be tooth-shaped.

The top surface of the cap 30 is provided with a blocking plate 31, with at least two through holes 32 formed in the blocking plate 31. Here, the blocking plate 31 functions to block the knot of the cerclage suture, and the through holes 32 allow the cerclage suture to pass therethrough.

Further, the top surface of the cap 30 is provided with a hook ring 34 for hooking a drawing line.

Hereinbelow, the operational theory of the mitral valve cerclage annuloplasty apparatus according to the present invention will be described with reference to FIGS. 3 to 5.

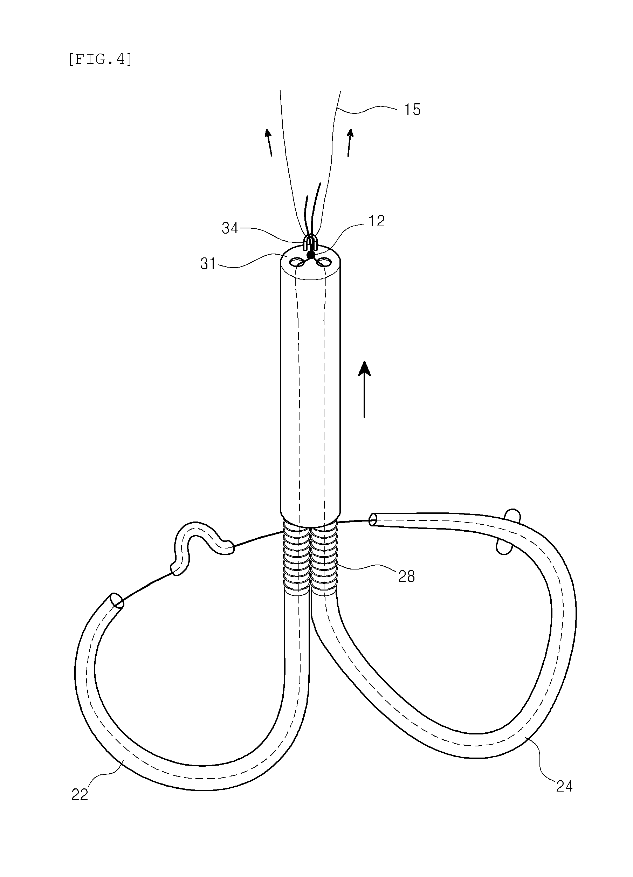

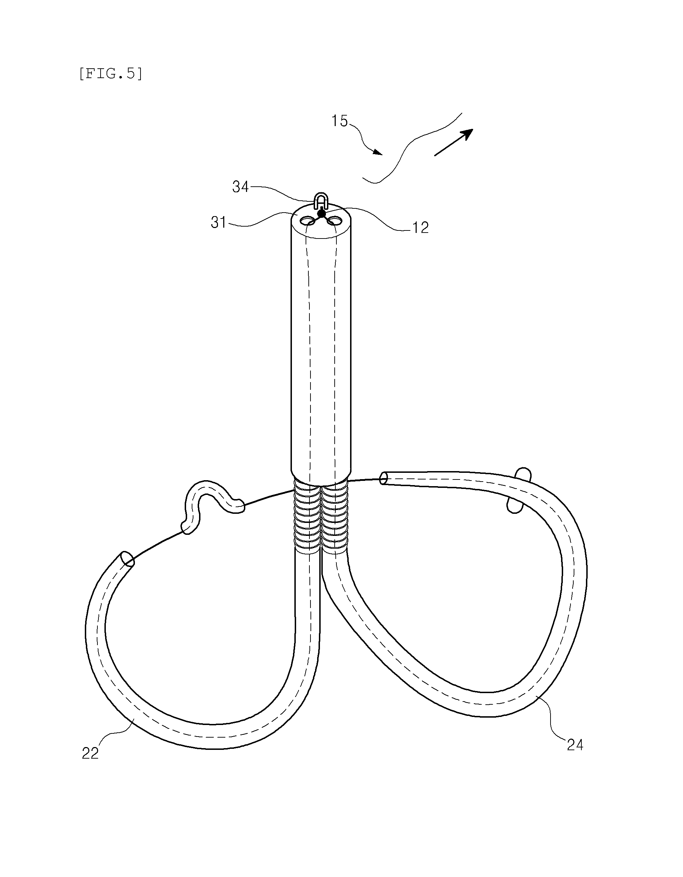

FIGS. 3 to 5 are views illustrating mitral valve cerclage annuloplasty performed using the mitral valve cerclage annuloplasty apparatus according to the preferred embodiment of the present invention, in which FIG. 3 illustrates a state in which a knot 12 is formed in a cerclage suture 10, FIG. 4 illustrates a state in which the cap 30 is raised upward from the state of FIG. 3 using a drawing line 15 and the knot 12 is blocked by the blocking plate 31 of the cap 30, and FIG. 5 illustrates a state in which a portion of the cerclage suture 10 is cut at a location above the knot 12 and the drawing line 15 is removed.

First, during mitral valve cerclage annuloplasty, the mitral annulus of the human body is tightened using a cerclage suture and the mitral valve cerclage annuloplasty apparatus of the present invention, and a knot 12 is formed in the cerclage suture using a knot delivery device disclosed in the Korean patent application (Korean Patent Application No. 2009-0080708) filed by the inventor of the this invention. When the knot 12 is formed in the cerclage suture, the knot 12 does not come into close contact with the top surface of the cap 30, so the cerclage suture is not tensioned over a predetermined tension (see FIG. 3).

Here, the drawing line 15 passes through the hook ring 34 of the cap 30 so as to be hooked thereby, and the opposite ends of the drawing line 15 are extended outside the body of a patient. The drawing line 15 is used to raise the cap 30 upward by drawing the cap 30.

As shown in FIG. 4, when a surgeon draws the drawing line 15 upward outside the body of a patient from the state shown in FIG. 3, the cap 30 is moved upward. In this case, the drawing line 15 is drawn so as to raise the cap 30 upward until the knot of the cerclage suture 12 is blocked by the blocking plate 31 of the cap and the cerclage suture is desirably tensioned. Here, since the catching depressions 28 of the tissue protective tube and the catching protrusions 35 of the cap are configured to allow easy separation of the tissue protective tube from the cap but to resist insertion of the tissue protective tube into the cap, the elevation of the cap 30 can be easily performed, but the cap 30 is free from undesirably moving downward even when the drawing line is not drawn.

As shown in FIG. 4, when the knot of the cerclage suture 12 is blocked by the blocking plate 31 of the cap and the cerclage suture is tensioned, a desired tension is continuously applied to the cerclage suture 10, so the cerclage suture 10 continuously tightens the mitral annulus of the patient. Accordingly, mitral regurgitation of the patient is corrected, and the success rate of mitral valve cerclage annuloplasty is increased.

In a state shown in FIG. 4, the cerclage suture 10 is cut using a cutter (not shown) at a location spaced apart from the knot 12, as shown in FIG. 5. After cutting the cerclage suture 10, free portions of the cerclage suture 10 are removed from the body of the patient. Thereafter, the drawing line 15 is removed from the body of the patient by pulling one end of the drawing line 15, so mitral valve cerclage annuloplasty is finished.

To use the mitral valve cerclage annuloplasty apparatus of the present invention during mitral valve cerclage annuloplasty, it is necessary to insert the tissue protective tube 20 into the cap 30 prior to starting mitral valve cerclage annuloplasty. However, in this case, due to the structure in that the catching depressions 28 of the tissue protective tube 20 and the catching protrusions 35 of the cap 30 are configured to resist insertion of the tissue protective tube into the cap, it is difficult to manually insert the tissue protective tube 20 into the cap 30.

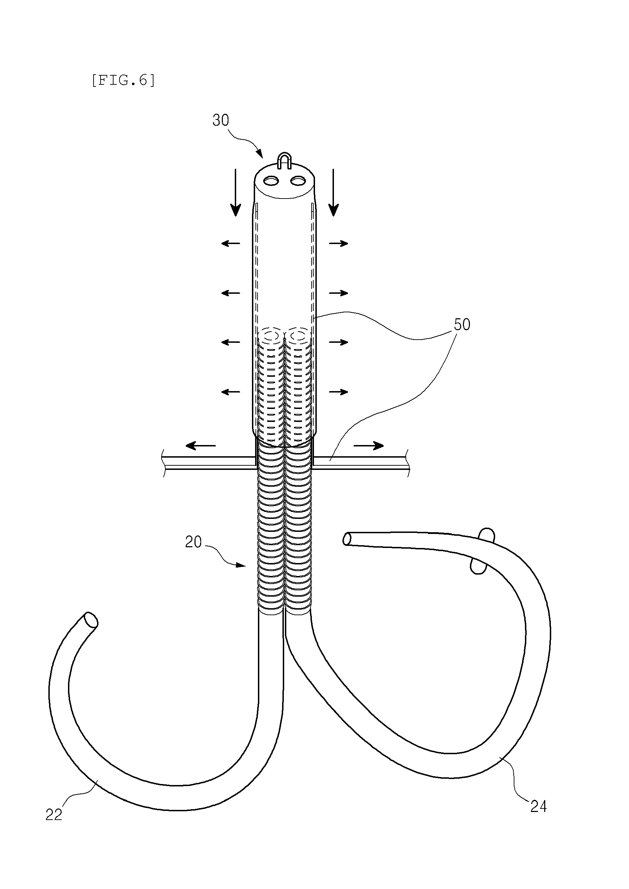

To accomplish the manual insertion of the tissue protective tube into the cap while overcoming the structural restriction, several methods may be proposed. One example of methods of inserting the tissue protective tube into the cap may be realized using expansion bars 50, as shown in FIG. 6.

FIG. 6 is a perspective view of the mitral valve cerclage annuloplasty apparatus according to the preferred embodiment of the present invention, in which an example of methods of combining the tissue protective tube with the cap is illustrated.

The expansion bars 50 form an expansion tool that can expand the interior space of the cap 30 so as to allow the tissue protective tube 20 to be inserted into the cap 30. Here, the cap 30 may be made of a soft material or a hard material. When the cap is made of a soft material, the interior space of the cap 30 may be expanded using the expansion bars 50, so the tissue protective tube 20 can be inserted into the cap 30.

The expansion bars 50 may comprise three or four bars that are inserted into the cap. When the expansion bars 50 are moved inside the cap such that the expansion bars 50 recede from each other, the interior space of the cap 30 is expanded, so the tissue protective tube 20 can be easily inserted into the cap 30.

After the tissue protective tube 20 is inserted into the cap 30, the expansion bars 50 are moved near to each other so as to be collected at their original positions. Thereafter, the expansion bars 50 are removed from the cap, so the combination of the cap 30 with the tissue protective tube 20 will be accomplished.

In the present invention, the expansion bars 50 may be installed in an expansion device (not shown), and may be actuated by a motor or a cylinder actuator (not shown). Here, the expansion device functions as a device that drives the expansion bars in such a way that the expansion bars can be moved in directions in which the expansion bars recede from each other or come near to each other.

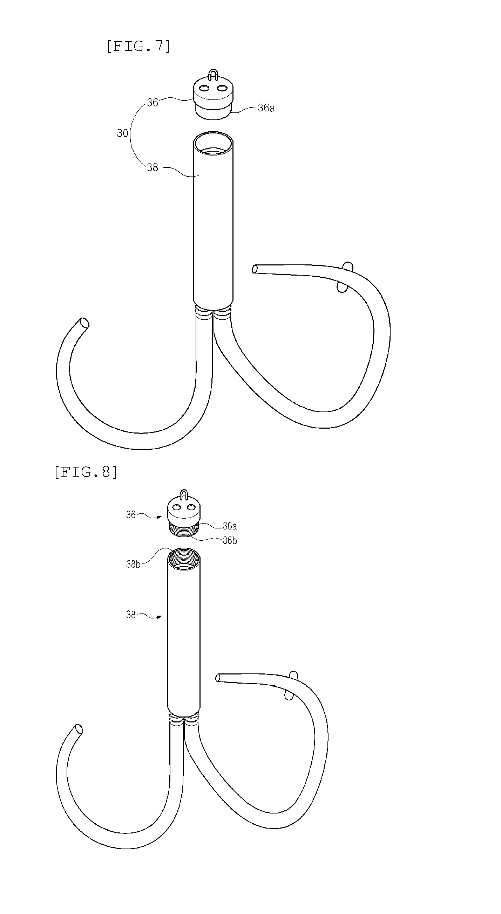

As another example of the methods of inserting the tissue protective tube 20 into the cap 30, the cap 30 may comprise two parts that are a body part 38 and a plug part 36 which are configured such that they can be separated from each other and can be combined with each other, as shown in FIG. 7.

As illustrated in FIG. 7, the cap 30 has a separable/combinable structure that includes the body part 38 and the plug part 36 which are configured to be separated from each other and to be combined with each other. The lower end of the plug part 36 is provided with an insert step 36a that can be inserted into and tightened to the body part. In other words, it is possible to separate the plug part 36 from the body part 38, and to combine the plug part 36 with the body part 38 by inserting the insert step 36a of the plug part into the body part 38.

To combine the tissue protective tube 20 with the cap 30, the plug part 36 is separated from the body part 38, and then, the upper part of the tissue protective tube is inserted into the upper part of the body part of the cap. After inserting the tissue protective tube 20 into the cap 30 as described above, the plug part 36 of the cap 30 is inserted into the body part 38, thereby accomplishing the combination of the tissue protective tube 20 with the cap 30. Here, to realize firm combination of the plug part with the body part, an adhesive may be used.

Of course, the firm combination of the plug part with the body part may be realized using another structure.

FIG. 8 illustrates another embodiment of the cap 30. As shown in the drawing, the body part 38 and the plug part 36 may be configured to have another separable/combinable structure, in which the outer circumferential surface of the insert step 36a of the plug part 36 is provided with external threads 36b and the inner circumferential surface of the body part 38 is provided with internal threads 38b corresponding to the external threads of the plug part. In this case, the plug part 36 can be combined with the body part 38 by being tightened thereto through screw-type engagement.

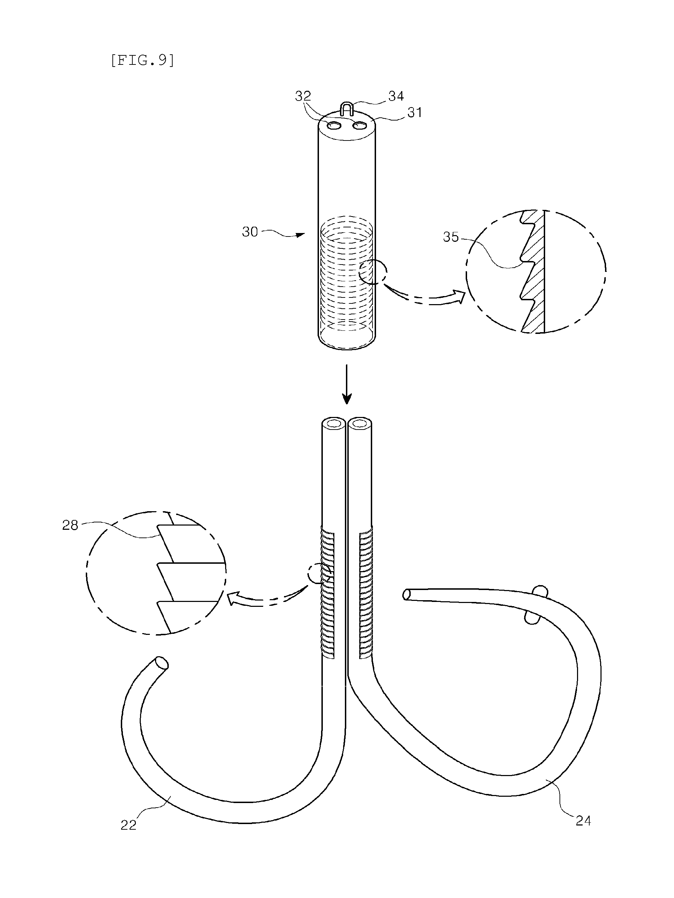

FIG. 9 is a perspective view of a mitral valve cerclage annuloplasty apparatus according to still another preferred embodiment of the present invention, in which the shape of catching depressions of the tissue protective tube is altered.

As shown in FIG. 9, the catching depressions 28 of the tissue protective tube may be partially formed within a predetermined range of a longitudinal stem part of the tissue protective tube without being totally formed on the stem part of the tissue protective tube. Particularly, the catching depressions 28 may be formed on opposite surfaces of the tissue protective tube 20 in lengthwise directions of the tissue protective tube. As shown in the drawing, the catching protrusions 35 of the cap 30 may be partially formed around the inner circumferential surface of the cap within a predetermined portion without being totally formed around the inner circumferential surface. Here, when the catching depressions 28 are formed on the opposite surfaces of the tissue protective tube 20 as described above, blood can easily flow after finishing the mitral valve cerclage annuloplasty.

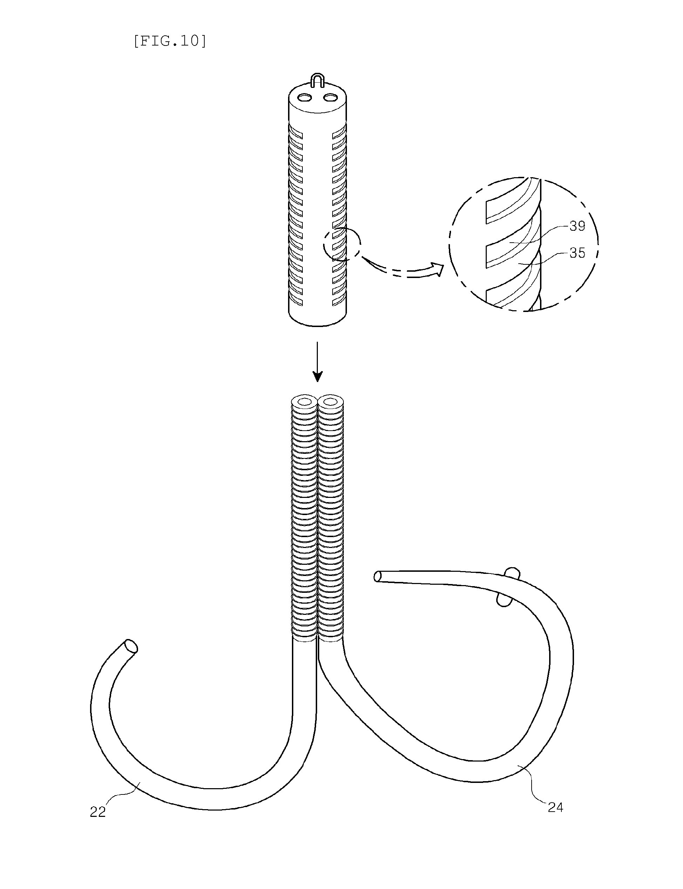

FIG. 10 is a perspective view of a mitral valve cerclage annuloplasty apparatus according to yet another preferred embodiment of the present invention, in which the shape of catching protrusions of the cap is altered.

As shown in FIG. 10, the catching protrusions 35 of the cap 30 may be formed by regularly spaced grooves 39.

Although the preferred embodiment(s) of the present invention have(has) been disclosed for illustrative purposes, those skilled in the art will appreciate that various modifications, additions and substitutions are possible, without departing from the scope and spirit of the invention as disclosed in the accompanying claims.

### [Industrial Applicability]

The present invention relates to a mitral valve cerclage annuloplasty apparatus which is used during mitral valve cerclage annuloplasty performed on mitral valve cerclage patients with mitral regurgitation, and which is efficiently applied to industrial fields that are required to safely and efficiently maintain a final appropriate tension of a cerclage suture required during mitral valve cerclage annuloplasty.

## Claims

1. A mitral valve cerclage annuloplasty apparatus, comprising: a tissue protective tube (20) including a coronary sinus tube (22) that is a hollow cylindrical tube used to protect coronary sinus tissues, a tricuspid valve tube (24) that is a hollow cylindrical tube used to commonly protect tricuspid valve tissues and interventricular septum tissues, and a stem part (26) which is formed by respective extensions of both the coronary sinus tube and the tricuspid valve tube and in which the coronary sinus tube and the tricuspid valve tube are integrated with each other, with catching depressions (28) formed on an outer circumferential surface of the stem part (26); and a cap (30) having a hollow structure so as to allow the tissue protective tube to be inserted into the cap, with a blocking plate (31) provided on a top surface of the cap (30), at least two through holes (32) formed in the blocking plate (31), and catching protrusions (35) formed on the cap (30) so as to correspond to the catching depressions of the tissue protective tube, wherein the catching depressions (28) of the tissue protective tube and the catching protrusions (35) of the cap are configured to allow easy separation of the tissue protective tube from the cap but to resist insertion of the tissue protective tube into the cap.

2. The mitral valve cerclage annuloplasty apparatus as set forth in claim 1, wherein the top surface of the cap (30) is provided with a hook ring (34) for hooking a line.

3. The mitral valve cerclage annuloplasty apparatus as set forth in claim 1, wherein the catching depressions of the tissue protective tube or the catching protrusions (35) of the cap are configured to be tooth-shaped.

4. The mitral valve cerclage annuloplasty apparatus as set forth in claim 1, wherein the catching depressions of the tissue protective tube is configured to be tooth-shaped and the catching protrusions of the cap are formed by regularly spaced grooves.

5. The mitral valve cerclage annuloplasty apparatus as set forth in claim 1, wherein the cap (30) includes a body part (38) and a plug part (36) that is placed on an upper end of the body part, with an insert step (36a) provided on a lower end of the plug part (36) so as to be inserted into the body part.

6. The mitral valve cerclage annuloplasty apparatus as set forth in claim 5, wherein an outer circumferential surface of the insert step of the plug part is provided with threads (36b) and an inner circumferential surface of the body part is provided with threads (38b) corresponding to the threads of the plug part.

7. The mitral valve cerclage annuloplasty apparatus as set forth in claim 1, wherein the catching depressions (28) of the tissue protective tube (20) are formed on opposite surfaces of the tissue protective tube (20) in lengthwise directions of the tissue protective tube.
